Europäisches Patentamt

European Patent Office

Office européen des brevets

(11) Numéro de publication : **0 477 244 B1**

(12) **FASCICULE DE BREVET EUROPEEN**

(19)

(45) Date de publication du fascicule du brevet :
**12.01.94 Bulletin 94/02**

(21) Numéro de dépôt : **90909447.6**

(22) Date de dépôt : **13.06.90**

(86) Numéro de dépôt international :
**PCT/FR90/00417**

(87) Numéro de publication internationale :
**WO 90/15587 27.12.90 Gazette 90/29**

(51) Int. Cl.⁵ : **A61K 6/00,** A61K 6/097

(54) **MATERIAU D'INSERTION UTILISE POUR ELARGIR LE SILLON GINGIVAL.**

(30) Priorité : **13.06.89 FR 8907812**

(43) Date de publication de la demande :
**01.04.92 Bulletin 92/14**

(45) Mention de la délivrance du brevet :
**12.01.94 Bulletin 94/02**

(84) Etats contractants désignés :
**AT BE CH DE DK ES FR GB IT LI NL SE**

(56) Documents cités :
**EP-A- 0 092 329
DE-A- 3 736 155
DE-A- 3 737 552
US-A- 4 468 202**

(73) Titulaire : **LESAGE, Patrick
9, rue Constantine
F-35400 Saint-Malo (FR)**

(72) Inventeur : **LESAGE, Patrick
9, rue Constantine
F-35400 Saint-Malo (FR)**

(74) Mandataire : **Hubert, Philippe et al
Cabinet Beau de Loménie 158, rue de
l'Université
F-75340 Paris Cédex 07 (FR)**

## Description

La présente invention a pour objet un matériau d'insertion utile pour élargir le sillon gingival, sensiblement sans saignement ou suintement.

On sait que le sillon gingival est un espace virtuel, se situant entre la dent et la gencive, qu'il est nécessaire d'élargir par exemple préalablement à la prise d'une empreinte ou encore pour la réalisation de soins divers, comme par exemple des collages, des scellements ou le traitement d'une carie en zone sous-gingival, afin d'obtenir un champ opératoire propre et sec.

Les techniques actuellement connues pour élargir le sillon gingival peuvent être réparties en deux groupes selon que l'élargissement est obtenu par éviction ou rétraction gingivale.

L'éviction gingivale est réalisée soit à l'aide d'un bistouri électrique soit à l'aide d'une fraise diamantée mue par une turbine (curetage rotatif).

Lorsqu'on utilise un bistouri électrique, on réalise une incision en partant du sommet du feston gingival et en se dirigeant vers le fond du sillon gingival encore dénommé sulcus. Les tissus incisés sont éliminés.

Bien que conduisant à un élargissement du sillon gingival avec une hémostase convenable, cette méthode est mutilante et douloureuse et nécessite généralement une anesthésie locale. Elle entraîne en outre une rétraction de la gencive.

Lorsqu'on utilise une fraise diamantée, l'éviction gingivale est réalisée en dilacérant la gencive marginale par l'action de la fraise, à laquelle on imprime un mouvement à l'intérieur du sulcus pour réaliser un biseau au niveau de la limite de la préparation.

Cette méthode qui n'est utilisée que dans certains cas spécifiques présente les mêmes inconvénients que celle décrite précédemment en référence au bistouri électrique. Le saignement est plus important et nécessite une hémostase secondaire.

Les procédés de rétraction gingivale se caractérisent par la mise en place dans le sillon gingival d'un matériau d'insertion.

La méthode la plus courante consiste à utiliser un cordonnet de coton toronné ou tressé, imbibé ou non d'une solution destinée à favoriser la rétraction.

Ce cordonnet est inséré à l'aide d'un instrument métallique à double coudage dans le sulcus au-delà de la limite de préparation, avant ou après la taille de la dent suivant les méthodes et le type de limite recherchés.

Cette méthode souffre cependant de divers inconvénients.

Tout d'abord, la mise en place du cordonnet sur tout le pourtour de la dent est délicate.

En outre, ce procédé est relativement douloureux et nécessite généralement une anesthésie locale. On observe de plus une lésion fréquente de l'attache épithéliale et des hémorragies ou suintement lors du retrait du cordonnet pour la prise d'empreinte.

Une autre méthode de rétraction gingivale consiste à utiliser, comme matériau d'insertion, un anneau réalisé à l'aide d'une matière spongieuse pouvant être imprégnée de solutions diverses (hémostatique, astringent, etc.).

Les résultats et inconvénients de cette méthode sont identiques à ceux décrits précédemment en référence à l'utilisation d'un cordonnet en coton.

Une troisième méthode de rétraction gingivale consiste à mettre en place sur la dent préparée une coiffe réalisée en un matériau spongieux pouvant être imprégné de solutions diverses et assurant la rétraction par l'application d'une pression occlusale.

Bien que rapide et de mise en oeuvre aisée, cette méthode est peu précise et n'assure pas une rétraction suffisante. Elle n'est donc actuellement utilisée que pour assurer l'hémostase après une procédure d'éviction gingivale.

Enfin, il a également été proposé d'utiliser, comme matériaux d'insertion, des compositions, dont certaines sont utilisées pour la prise d'empreinte, se présentant sous la forme d'un produit fluide injectable susceptible de durcir soit par réaction chimique (mélange préalable avec un durcisseur), soit par gonflement physique (absorption d'eau).

Cet état de la technique est notamment illustré par les documents DE-A-3.737.552 ; DE-A-3.736.155 et EP-A-0.092.329.

Cependant, de telles compositions fluides sont très difficiles à mettre en place dans le sillon gingival qui est un espace virtuel et dont l'élargissement ne peut être obtenu que sous l'effet d'une force relativement élevée.

En outre, il est impossible, à l'aide de ces compositions "durcissantes", d'obtenir une rétraction contrôlée de la gencive et la mise en oeuvre de telles compositions est relativement lente.

La présente invention a donc pour but de résoudre les inconvénients précités en proposant un nouveau matériau d'insertion permettant l'obtention d'une rétraction large et régulière sur tout le pourtour de la dent,

EP 0 477 244 B1

qui n'entraîne aucune lésion notable de la gencive marginale et qui puisse être mise en oeuvre facilement sans saignement ou suintement.

La solution conforme à la présente invention, pour résoudre ce problème technique consiste en un matériau d'insertion utilisé pour élargir le sillon gingival, sensiblement sans saignement ou suintement, caractérisé en ce qu'il se présente sous forme d'une pâte biocompatible et injectable à usage externe, de préférence hydrophile et dont les propriétés rhéologiques sont telles qu'elles permettent de s'opposer à la force tendant à appliquer la gencive marginale contre la dent sans lésion des structures anatomiques voisines.

Selon une caractéristique particulière, cette pâte présente une viscosité supérieure à environ 13000 Pascals.seconde.

Un tel matériau peut être facilement injecté entre la dent et la gencive et sa consistance rend possible l'obtention d'un élargissement parfaitement contrôlé du sillon gingival.

La viscosité définie précédemment est une viscosité plastique mesurée à 20°C.

Selon une autre caractéristique, cette pâte présente une valeur de seuil d'écoulement supérieure à environ 4500 Newtons par m2.

Selon un mode de réalisation actuellement préféré, ce matériau comprend de l'argile, en particulier une argile kaolinique micronisée (argile blanche), et de l'eau.

Ce matériau comprend éventuellement en outre des agents modifiant les propriétés organoleptiques et chromatiques et/ou un agent astringent et/ou un agent antiseptique.

L'utilisation d'un tel matériau conforme à l'invention permet l'élaboration d'une méthode de rétraction gingivale perfectionnée qui ne souffre pas des divers inconvénients des méthodes utilisées jusqu'à présent. En effet, cette méthode est peu agressive puisqu'elle n'entraîne pratiquement pas de saignement ou de suintement et est par conséquent indolore.

En outre, la pression excercée sur les tissus gingivaux étant uniquement fonction de la viscosité du matériau d'insertion, il n'y a pas de lésion notable de la gencive marginale et la rétraction se trouve ainsi réversible.

L'invention sera mieux comprise et d'autres buts, caractéristiques et avantages de celle-ci apparaîtront plus clairement à la lumière de la description explicative qui va suivre, faite en référence aux dessins schématiques annexés donnés uniquement à titre illustratif et dans lesquels :

- la figure 1 est une vue schématique en coupe transversale montrant une dent ;
- la figure 2 est une vue en coupe transversale du détail A de la figure 1 ; et
- la figure 3 est une vue en coupe transversale d'une seringue permettant l'insertion par injection d'un matériau conforme à l'invention.

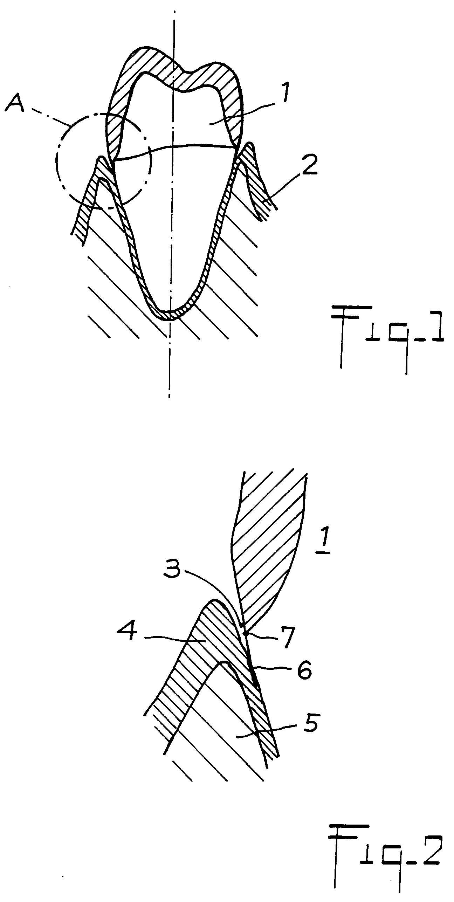

On a donc représenté schématiquement à la figure 1 une dent 1 et la gencive 2 entourant sa base.

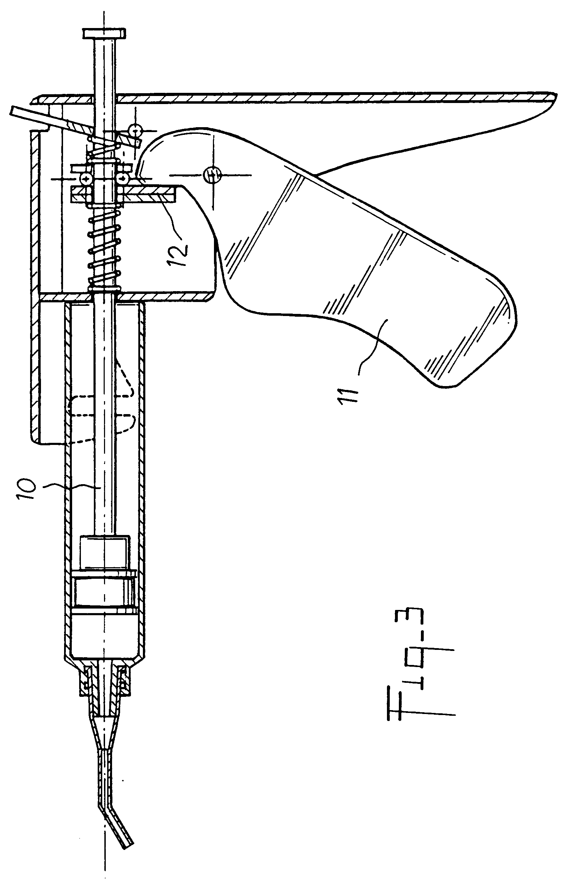

La figure 2 qui est une vue agrandie du détail A de la figure 1 montre le sillon gingival 3 compris entre la dent 1 et la gencive marginale 4.

Le repère 5 représente l'os alvéolaire et le repère 6 l'attache épithéliale.

On a représenté par le repère 7 la limite de préparation de la dent qui se situe entre l'attache épithéliale 6 et la partie supérieure de la gencive marginale 4.

La partie hachurée sur la figure 2 représente la portion scellée destinée à former prothèse.

L'originalité de la présente invention consiste à utiliser un matériau d'insertion se présentant sous la forme d'une pâte dont les propriétés rhéologiques sont telles qu'elles permettent de s'opposer à la force tendant à appliquer la gencive marginale contre la dent sans lésion des structures anatomiques voisines.

Cette pâte doit présenter une biocompatibilité parfaite et être injectable sous faible diamètre par exemple 0,3 mm à 1,5 mm.

On peut utiliser de nombreux produits présentant ces propriétés, comme par exemple les argiles, des cires ou d'autres matériaux biocompatibles.

Selon un premier mode de réalisation de l'invention, on utilisera un matériau comprenant de la farine d'algues, notamment laminaire et en particulier laminaria digitata.

La viscosité de ce matériau à base d'algues pourra être ajustée dans les gammes préférées précitées à l'aide d'eau.

Selon un autre mode de réalisation actuellement préféré de l'invention, on utilisera un matériau comprenant de l'argile en particulier une argile kaolinique micronisée, et la encore, la viscosité peut être facilement ajustée dans les gammes préférées à l'aide d'eau.

Par ailleurs, on pourra utiliser divers agents additionnels comme des agents modifiant les propriétés organoleptiques et chromatiques, des agents astringents et/ou antiseptiques.

A titre d'exemple, des agents modifiant les propriétés organoleptiques peuvent être constitués par ou contenir au moins l'un des composants suivants :

- huile essentielle de citron vert, orange, menthe,
- bicarbonate de soude,

3

- eau oxygénée 10 volumes.

A titre d'exemple, des agents modifiant les propriétés chromatiques peuvent être constitués par ou contenir au moins l'un des composants suivants :

- eau oxygénée 10 volumes,
- oxyde de zinc,
- argile blanche,
- colorants alimentaires divers.

A titre d'exemple, des agents astringents peuvent être constitués par ou contenir au moins l'un des composants suivants :

- chlorure d'aluminium,
- chlorure ferrique,
- sulfate ferrique,
- aluns (sulfate double d'aluminium et de potassium).

A titre d'exemple, des agents hémostatiques peuvent être constitués par ou contenir au moins l'un des composants suivants :

- alginate de calcium,
- oxyquinol,
- eau oxygénée 10 volumes.

Enfin, à titre d'antiseptique, on pourra utiliser du digluconate de chlorexidine.

D'une façon générale les produits hydrophiles conduisent à de meilleurs résulats que les produits hydrophobes.

Une composition actuellement préférée de matériau d'insertion conforme à l'invention est la suivante :
- argile blanche (argile kaolinique micronisée)     65 à 70 % en poids
- chlorure d'aluminium     3,6 à 6,8 % en poids
- $H_2O$     24 à 27 % en poids
- huile essentielle     0,33 % en poids
- colorant E 102 (sel trisodique de l'acide (sulfo-4' phénylazo-1')4 [sulfo-4 phényl -1 hydroxy-5 pyrazolecarboxylique-3]) + E 131 (sel calcique de l'acide disulfonique de l'anhydride m-hydroxytétraéthyl diamino triphényl carbinol).     1 % en poids

On a donné aux exemples 1 à 7 plusieurs compositions de matériau conforme à la présente invention.

EXEMPLE 1 :

argile blanche     : 66,75 %
chlorure d'aluminium     : 6,54 %
$H_2O$     : 25,36 %
huile essentielle citron     : 0,33 %
colorant (E 102 + E 131)     : 1,02 %

EXEMPLE 2 :

argile blanche     : 65,60 %
chlorure d'aluminium     : 6,83 %
$H_2O$     : 26,24 %
huile essentielle citron     : 0,33 %
colorant (E 102 + E 131)     : 1,00 %

EXEMPLE 3 :

argile blanche     : 69,83 %
chlorure d'aluminium     : 3,64
$H_2O$     : 25,14
huile essentielle citron     : 0,35
colorant (E 102 + E 131)     : 1,04 %

EXEMPLE 4 :

farine d'algues     : 46 %

eau            : 54 %

EXEMPLE 5 :

```
farine d'algue micronisée laminaria digitata : 46,1  %
eau distillée                                 : 53,85 %
digluconate de chlorexidine                   :  0,05 %
```

EXEMPLE 6 :

```
farine d'algue micronisée laminaria digitata : 45,63 %
eau oxygénée 10 volumes                       : 45,63 %
huile essentielle orange                      :  1,14 %
oxyde de zinc                                 :  7,55 %
digluconate de chlorexidine                   :  0,05 %
```

EXEMPLE 7 :

```
farine d'algue micronisée laminaria digitata : 34,08 %
argile blanche                               : 19,47 %
eau oxygénée 10 volumes                      : 43,82 %
bicarbonate de soude                         :  2,43 %
huile essentielle orange                     :  0,20 %
```

Les matériaux ainsi préparés à base d'algues se comportent comme des plastiques de Bingham. Ils présentent une viscosité plastique à 20°C comprise entre environ 13000 et environ 30000 Pascals.seconde, et un seuil d'écoulement d'environ 5000 Newtons/m 2 (mesures réalisées en fluage à l'aide d'un rhéomètre à contrainte imposée muni d'un dispositif cône-plan à 20°C (cône de diamètre 1,5 cm, angle de 4°).

Les matériaux à base d'argile sont caractérisés par les valeurs suivantes :

- seuil d'écoulement supérieur à environ 4500 Newtons/m2 ;
- viscosité dynamique supérieure à environ 27000 Pascals.seconde (sous une contrainte de 9000 Newtons/m2 et une vitesse de 0,3 s.$^{-1}$).

Un matériau conforme à la présente invention peut être inséré dans le sillon gingival par injection au moyen d'une aiguille cylindrique ou conique dont L'extrémité présente une lumière d'un diamètre compris entre environ 0,3 et 1,5 mm.

Avantageusement, cette aiguille présentera un coudage d'environ 30°.

On utilisera une aiguille stérilisable ou à usage unique pouvant être changée après chaque opération.

Le diamètre de l'aiguille sera sélectionné selon le type de préparation et de limite ainsi que selon l'importance du sillon gingival. Ainsi, on utilisera une aiguille d'un diamètre d'environ 1 à 1,5 mm pour un congé large, et de 0,5 mm pour une limite en biseau.

La profondeur du Sulcus, l'épaisseur et la tonicité de la gencive marginale sont variables sur le pourtour d'une même dent, de sorte qu'il est souhaitable de pouvoir contrôler l'importance de la rétraction recherchée en chaque point.

L'injection d'un produit pâteux permet, en déposant plus ou moins de produit, d'obtenir une rétraction large et régulière sur tout le pourtour de la dent.

L'injection du produit pâteux est effectuée dans le sillon gingival en prenant appui sur la dent et en n'exerçant aucune pression avec l'aiguille sur les tissus.

Comme on le comprend, la pression excercée sur les tissus gingivaux est uniquement fonction de la viscosité du produit.

On obtient ainsi une rétraction large et régulière sur tout le pourtour de la dent.

On a représenté à la figure 3 un mode de réalisation actuellement préféré d'un dispositif d'injection permettant la mise en place du matériau d'insertion conforme à l'invention. En effet, le produit utilisé ayant une viscosité relativement élevée, la pression développée dans une seringue classique n'est pas suffisante.

On peut donc utiliser une seringue telle que celle représentée à la figure 3 dont la partie active 10 est entraînée par une plaque 12, elle-même mue par la poignée 11, transmettant en la multipliant par effet de levier la force développée entre les doigts et la paume de la main.

Le matériau conforme à l'invention peut être éliminé sous l'action d'un spray air et eau sans intervention d'instrument préalalement à la prise d'empreinte. On évite ainsi tout risque de léser les tissus.

Il est enfin à noter que le matériau conforme à l'invention peut être également utilisé après une procédure d'éviction gingivale par bistouri électrique ou curtage rotatif pour assurer l'hémostase et le maintien de l'élargissement du sillon gingival.

Comme on le comprend, l'utilisation du matériau conforme à la présente invention permet la mise en oeuvre d'une méthode de rétraction gingivale améliorée qui regroupe pour la première fois l'ensemble des avantages suivants :

- accessibilité aisée de la limite de préparation ;
- absence de lésion des tissus ;
- rétraction réversible ;
- mise en oeuvre rapide, aisée et indolore.

## Revendications

1. Matériau d'insertion utilisé pour élargir le sillon gingival, sensiblement sans saignement ou suintement, notamment afin de prendre une empreinte pour la réalisation d'une prothèse de dent, caractérisé en ce qu'il se présente sous forme d'une pâte biocompatible et injectable à usage externe, de préférence hydrophile, dont la viscosité plastique mesurée à 20°C est supérieure à 13000 Pascals.seconde.

2. Matériau selon la revendication 1, caractérisé en ce qu'il se présente sous forme d'une pâte dont le seuil d'écoulement est supérieur à 4500 Newtons/m$^2$.

3. Matériau selon l'une quelconque des revendications 1 ou 2, caractérisé en ce qu'il comprend de l'argile, notamment de l'argile blanche.

4. Matériau selon l'une quelconque des revendications 1 ou 2, caractérisé en ce qu'il comprend de la farine d'algues, notamment de laminaire et en particulier Lamminaria digitata, et de l'eau.

5. Matériau selon la revendication 3 ou 4, caractérisé en ce qu'il comprend en outre des agents modifiant les propriétés organoleptiques, comme par exemple de l'huile essentielle de citron vert, orange, menthe ; du bicarbonate de soude ou de l'eau oxygénée à 10 volumes (solution aqueuse 3 %).

6. Matériau selon la revendication 3, 4 ou 5, caractérisé en ce qu'il comprend en outre des agents modifiant les propriétés chromatiques, comme par exemple l'eau oxygénée à 10 volumes (solution aqueuse 3 %), l'oxyde de zinc, l'argile blanche ou des colorants alimentaires divers, et éventuellement un agent astringent et/ou antiseptique.

7. Matériau selon la revendication 4, caractérisé en ce qu'il présente la composition en poids suivante :
   farine d'algues         : 46 %
   eau                     : 54 %

8. Matériau selon l'une des revendications 4, 5 ou 6, caractérisé en ce qu'il présente la composition en poids suivante :

```
farine d'algue micronisée laminaria digitata : 45,63 %
eau oxygénée 10 volumes (solution aqueuse 3 %)   : 45,63 %
huile essentielle orange                          :  1,14 %
oxyde de zinc                                     :  7,55 %
digluconate de chlorexidine                      :  0,05 %
```

9. Matériau selon l'une des revendications 4, 5 ou 6, caractérisé en ce qu'il présente la composition en poids suivante :

```
farine d'algue micronisée laminaria digitata : 34,08 %
argile blanche                               : 19,47 %
eau oxygénée 10 volumes (solution aqueuse 3 %) : 43,82 %
bicarbonate de soude                         :  2,43 %
huile essentielle orange                     :  0,20 %
```

10. Matériau selon la revendication 3, 5 ou 6, caractérisé en ce qu'il présente la composition en poids suivante :
    - argile blanche (argile kaolinique micronisée)     65 à 70 % en poids
    - chlorure d'aluminium     3,6 à 6,8 % en poids
    - $H_2O$     24 à 27 % en poids
    - huile essentielle     0,33 % en poids
    - colorant E 102 (sel trisodique de l'acide (sulfo-4' phényl-azo-1')-4 [sulfo-4 phényl -1 hydroxy-5 pyrazolecarboxylique-3]) + E 131 (sel calcique de l'acide disulfonique de l'anhydride m-hydroxytétraéthyl diamino triphényl carbinol).     1 % en poids

**Patentansprüche**

1. Einsetzmaterial zur Vergrößerung des Zahnfleischsulcus, ohne merkliches Bluten oder Nässen, insbesondere zur Abnahme eines Abdrucks zur Herstellung einer zahnprothese, dadurch gekennzeichnet, daß es in Form einer bioverträglichen und bei äußerer Anwendung injizierbaren Paste, vorzugsweise von hydrophiler Beschaffenheit, vorliegt, deren bei 20°C gemessene plastische Viskosität mehr als 13000 Pascal.sec beträgt.

2. Material nach Anspruch 1, dadurch gekennzeichnet, daß es in Form einer Paste vorliegt, deren Fließgrenze mehr als 4500 Newton/m$^2$ beträgt.

3. Material nach einem der Ansprüche 1 oder 2, dadurch gekennzeichnet, daß es Ton, insbesondere weißen Ton, enthält.

4. Material nach einem der Ansprüche 1 oder 2, dadurch gekennzeichnet, daß es Algenmehl, insbesondere von Lamminaria und ganz besonders von Lamminaria digitata, sowie Wasser enthält.

5. Material nach Anspruch 3 oder 4, dadurch gekennzeichnet, daß es außerdem Mittel zur Modifikation der organoleptischen Eigenschaften, z.B. ätherisches Öl der grünen Zitrone, Orange oder Minze; Natriumbicarbonat und Wasserstoffperoxid (10 Volumina; 3 % wäßrige Lösung) enthält.

6. Material nach Anspruch 3, 4 oder 5, dadurch gekennzeichnet, daß es ferner Mittel zur Modifikation der Farbeigenschaften, z.B. Wasserstoffperoxid (10 Volumina; 3 % wäßrige Lösung), Zinkoxid, weißen Ton oder verschiedene Lebensmittelfarbstoffe, und gegebenenfalls ein adstringierendes und/oder antiseptisches Mittel enthält.

7. Material nach Anspruch 4, dadurch gekennzeichnet, daß es folgende, auf das Gewicht bezogene Zusammensetzung aufweist:
Algenmehl:    46 %
Wasser :    54 %

8.  Verfahren nach einem der Ansprüche 4, 5 oder 6, dadurch gekennzeichnet, daß es folgende, auf das Gewicht bezogene Zusammensetzung aufweist:

```
mikronisiertes Algenmehl von Laminaria digitata:     45,63 %

Wasserstoffperoxid (10 Volumina;
     3 % wäßrige Lösung                          :     45,63 %

etherisches Öl der Orange                        :      1,14 %

Zinkoxid                                          :      7,55 %

Chlorhexidindigluconat                           :      0,05 %
```

9.  Material nach einem der Ansprüche 4, 5 oder 6, dadurch gekennzeichnet, daß es folgende, auf das Gewicht bezogene Zusammensetzung aufweist:

```
mikronisiertes Algenmehl von Laminaria digitata:     34,08 %

weißer Ton                                       :     19,47 %

Wasserstoffperoxid (10 Volumina;
     3 % wäßrige Lösung)                          :     43,82 %

Natriumbicarbonat                                :      2,43 %

etherisches Öl der Orange                        :      0,20 %
```

10. Material nach Anspruch 3, 5 oder 6, dadurch gekennzeichnet, daß es folgende, auf das Gewicht bezogene Zusammensetzung aufweist:
    - weißer Ton (mikronisierter Kaolinton)        65 - 70 Gew.-%
    - Aluminiumchlorid        3,6 - 6,8 Gew.-%
    - $H_2O$        24 - 27 Gew.-%
    - etherisches Öl        0,33 Gew.-%
    - Farbstoff E 102 (Trinatriumsalz von 5-Hydroxy-1-(4-sulfophenyl)-4-(4-sulfophenylazo)-1 H-pyrazol-3-carbonsäure) + E 131 (Calciumsalz von Bis-(4-Diethylaminophenyl)-(2,4-disulfo-3-hydroxyphenyl-)-carbinolanhydrid).        1 Gew.-%

## Claims

1.  Insert material used for widening the gingival sulcus, substantially without bleeding or oozing, particularly in order to take an impression for making a dental prosthesis, characterized in that it takes the form of a biocompatible paste which is injectable for external use, preferably hydrophilic, whose plastic viscosity measured at 20°C is greater than 13000 Pascals.second.

2.  Material according to claim 1, characterized in that it takes the form of a paste whose flow threshold is greater than 4500 Newtons/m$^2$.

3.  Material according to any one of claims 1 or 2, characterized in that it includes clay, particularly China clay.

4.  Material according to any one of claims 1 or 2, characterized in that it includes flour of algae, particularly laminary and in particular laminaria digitata, and water.

5.  Material according to claim 3 or 4, characterized in that it further includes agents modifying the organoleptic properties, such as for example essential oil of lime, orange, mint; sodium bicarbonate or 10 vol. hydrogen peroxide (3% aqueous solution).

6. Material according to claim 3, 4 or 5, characterized in that it further includes agents modifying the chromatic properties, such as for example 10 vol. hydrogen peroxide (3% aqueous solution), zinc oxide, China clay or various alimentary colorants, and possibly an astringent and/or antiseptic agent.

7. Material according to claim 4, characterized in that it presents the following composition by weight:
flour of algae        46%
water                 54%

8. Material according to one of claims 4, 5 or 6, characterized in that it presents the following composition by weight:

```
micronized flour of algae
laminaria digitata        45.63%
10 vol. hydrogen peroxide
(3% aqueous solution)     45.63%
essential oil of orange    1.14%
zinc oxide                 7.55%
digluconate of chlorexidine 0.05%
```

9. Material according to one of claims 4, 5 or 6, characterized in that it presents the following composition by weight:

```
micronized flour of algae
laminaria digitata        34.08%
China clay                19.47%
10 vol. hydrogen peroxide
(3% aqueous solution)     43.82%
sodium bicarbonate         2.43%
essential oil of orange    0.20%
```

10. Material according to claim 3, 5 or 6, characterized in that it presents the following composition by weight:
   - China clay (micronized kaolin)    65 to 70% by weight
   - aluminium chloride    3.6 to 6.8% by weight
   - $H_2O$    24 to 27% by weight
   - essential oil    0.33% by weight
   - colorant E 102 (trisodic salt of 4-(4'-sulfophenyl 1'-azo) [4-sulfophenyl 5-hydroxy 3-pyrazolcarboxylic] acid) + E 131 (calcic salt of disulfonic acid of m-hydroxytetraethyldiamino triphenyl carbinol anhydride)    1% by weight.

Fig.1

Fig.2

Fig.3